# EUROPEAN PATENT APPLICATION

(11) **EP 4 681 741 A1**
(43) Date of publication of application: **21.01.2026**
(21) Application number: 24770043.8
(22) Date of filing: 18.03.2024
(51) Int. Cl.: A61K 51/04, A61K 101/02

(54) **USE OF POSITRON MYOCARDIAL FATTY ACID METABOLISM IMAGING AGENT AND POSITRON 18F-FDG MYOCARDIAL GLUCOSE IMAGING AGENT IN PET COMBINED IMAGING**

(30) Priority: 16.03.2023 CN 202310255399
(71) Applicant: Beijing Sinotau International Pharmaceutical Technology Co., Ltd., Beijing 100176 (CN)
(72) Inventor: XU, Bailing, Beijing 100176 (CN); TANG, Yanmin, Beijing 100176 (CN); XU, Xinsheng, Beijing 100176 (CN)
(74) Representative: Lavoix
(86) International application number: PCT/CN2024/082193
(87) International publication number: WO 2024/188353

(57) **Abstract**

A use of a positron myocardial fatty acid metabolism imaging agent and a positron ¹⁸F-FDG myocardial glucose imaging agent in PET combined imaging.

## Description

### TECHNICAL FIELD

The present application relates to the field of medical reagents, and in particular to the use of a positron myocardial fatty acid metabolism imaging agent and a positron myocardial glucose imaging agent ¹⁸F-FDG for PET combined imaging.

### BACKGROUND ART

Assessment of viable myocardium is a necessary step before coronary revascularization for patients with acute myocardial infarction, old myocardial infarction, and total coronary occlusion. Nuclear medicine positron myocardial metabolic imaging is considered the most effective method for the assessment of viable myocardium. However, the current method of myocardial metabolic imaging, which involves injecting ¹⁸F-FDG after regulating blood glucose, takes too long (1-4 hours), and imaging is often impossible for patients with insulin resistance. Hypoglycemia events often occur during the blood glucose regulation process, resulting in low clinical utility. The present patent application uses a combined ¹⁸F-FDG and positron myocardial fatty acid metabolism imaging under fasting conditions for the subjects to replace the existing blood glucose-regulated ¹⁸F-FDG myocardial metabolic imaging to evaluate viable myocardium and scarred myocardium. The objective of the present application is to propose a new technical solution that can avoid the various problems caused by blood glucose regulation while provide assessment of viable myocardium.

### SUMMARY OF THE INVENTION

To achieve the above objectives, the present application provides:
1. Use of a positron myocardial fatty acid metabolism imaging agent and a positron myocardial glucose imaging agent ¹⁸F-FDG for preparing a medicament for PET combined imaging, wherein,
   the chemical formula of the positron myocardial fatty acid metabolism imaging agent is as follows: 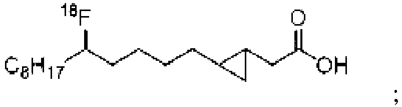
   the chemical formula of the positron myocardial glucose imaging agent ¹⁸F-FDG is: 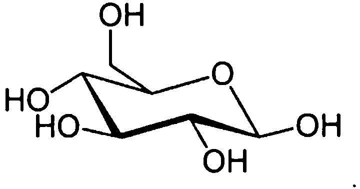
2. The use according to item 1, wherein the PET combined imaging is applied to a mammal.
3. The use according to item 2, wherein
   the positron myocardial fatty acid metabolism imaging agent and the positron myocardial glucose imaging agent ¹⁸F-FDG are administered to a subject on the same day,
   during administration, the subject is first administered with the myocardial glucose imaging agent ¹⁸F-FDG for imaging, followed by the administration of the positron myocardial fatty acid metabolism imaging agent for imaging. Imaging is performed approximately one hour after the injection of the ¹⁸F-FDG. Immediately after completion of this imaging, the positron myocardial fatty acid metabolism imaging agent is injected, and imaging is performed promptly thereafter,
      or
   the subject is first administered with the positron myocardial fatty acid metabolism imaging agent for imaging, followed by the myocardial glucose imaging agent ¹⁸F-FDG for imaging. Imaging is performed immediately after the injection of the positron myocardial fatty acid metabolism imaging agent. Upon completion of this imaging session, the myocardial glucose imaging agent ¹⁸F-FDG is injected, and imaging is performed nearly one hour after the injection,
   the subject needs to fast for at least 4 hours before sequentially administering the two imaging agents.
4. The use according to item 3, wherein, when administered to a subject on the same day, the subject is first administered with the myocardial glucose imaging agent ¹⁸F-FDG for imaging, followed by the positron myocardial fatty acid metabolism imaging agent for imaging,
   the ratio of injection dose (Unit: mCi or MBq) of the positron myocardial glucose imaging agent ¹⁸F-FDG to that of the positron myocardial fatty acid metabolism imaging agent is 1:x, and x≥1.
5. The use according to item 4, wherein x is 2 to 4.
6. The use according to item 3, wherein, when administered to a subject on the same day, the subject is first administered the positron myocardial fatty acid metabolism imaging agent for imaging, followed by administration of the positron myocardial glucose imaging agent ¹⁸F-FDG for imaging,
   the ratio of injection dose (Unit: mCi or MBq) of the positron myocardial fatty acid metabolism imaging agent to the ¹⁸F-FDG myocardial glucose imaging agent is 1:x, and x≥1.
7. The use according to item 6, wherein x is 2 to 4.
8. The use according to item 2, wherein the positron myocardial fatty acid metabolism imaging agent and the positron myocardial glucose imaging agent ¹⁸F-FDG are administered to the subject on two separate days,
   during administration, the subject is administered with the ¹⁸F-FDG myocardial glucose imaging agent for imaging on the first day, followed by administration of the positron myocardial fatty acid metabolism imaging agent for imaging on the second day, or
   during administration, the subject is administered with the positron myocardial fatty acid metabolism imaging agent for imaging on the first day, followed by administration of the ¹⁸F-FDG myocardial glucose imaging agent for imaging on the second day;
   the subject needs to fast for at least 4 hours before administering the imaging agents on two separate days.
9. The use according to item 2, wherein the PET images of the PET combined imaging are used in conjunction with myocardial perfusion images to assess the viable myocardium and scarred myocardium in the infarcted area, and the myocardial perfusion images are obtained from SPECT or PET myocardial perfusion imaging.
10. An imaging agent kit, comprising a positron myocardial fatty acid metabolism imaging agent and a positron ¹⁸F-FDG G myocardial glucose imaging agent;
   the chemical formula of the positron myocardial fatty acid metabolism imaging agent is as follows: 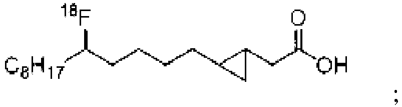
   the chemical formula of the positron myocardial glucose imaging agent ¹⁸F-FDG is:

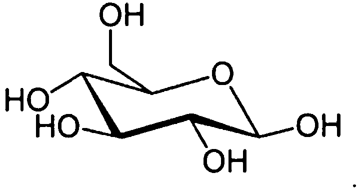

### BENEFICIAL TECHNICAL EFFECTS ACHIEVED BY THE TECHNICAL SOLUTION OF THE PRESENT APPLICATION

The solution of the present application can avoid the problems of low efficiency of blood glucose regulation in ¹⁸F-FDG myocardial metabolic imaging, poor image quality in patients with insulin resistance, and hypoglycemia events during blood glucose regulation, thereby improving the clinical utility of nuclear medicine-based myocardial metabolic imaging, allowing more myocardial infarction patients to obtain comprehensive viability assessment prior to being subjected to revascularization, and then decide on the necessity of undergoing major surgery.

### BRIEF DESCRIPTION OF THE DRAWINGS

Fig. 1a shows the SPECT myocardial perfusion images according to Example 1, and Figs. 1b-1c are the combined PET imaging results of ¹⁸F-FDG and XTR003.
Fig. 2a shows the SPECT myocardial perfusion images according to Example 2, and Figs. 2b-2c are the combined PET imaging results of ¹⁸F-FDG and XTR003.
Figs. 3a-3b are the combined PET imaging results of ¹⁸F-FDG and XTR003 according to Comparative Example 1.
Figs. 4a-4b are the combined PET imaging results of XTR003 and ¹⁸F-FDG according to Example 3.
Figs. 5a-5b are the combined PET imaging results of XTR003 and ¹⁸F-FDG according to Comparative Example 2.
Figs. 6a-6b are the combined PET imaging results of ¹⁸F-FDG and XTR003 according to Example 4.
Figs. 7a and 7b are the PET imaging results of ¹⁸F-FDG and XTR003 according to Comparative Example 3, respectively.
Figs. 8a-8b are the combined PET imaging results of ¹⁸F-FDG and XTR003 according to Comparative Example 4.
Figs. 9a-9b are the combined PET imaging results of XTR003 and ¹⁸F-FDG according to Comparative Example 5.

### DETAIL DESCRIPTION OF THE INVENTION

Specific embodiments of the present application will be described in more detail below with reference to the accompanying drawings. Although specific embodiments of the present application are shown in the accompanying drawings, it should be understood that the present application can be implemented in various forms and should not be construed as limited to the embodiments set forth herein. On the contrary, these embodiments are provided to enable a more thorough understanding of the present application and to fully convey the scope of the present application to those skilled in the art.

It should be noted that certain terms are used in the specification and claims to refer to specific components. Those skilled in the art will appreciate that they may use different terms to refer to the same component. The specification and claims do not use differences in nomenclature to distinguish components, but rather use differences in functions of components as the criteria for distinction. As mentioned throughout the specification and claims, "comprising" or "including" is an open-ended term and should be interpreted as "including but not limited to". The following description are preferred embodiments of the present application. However, these descriptions are for the purpose of explaining the general principles of the specification and are not intended to limit the scope of the present application. The protection scope of the present application shall be determined by the appended claims.

As used herein, "substantially free" with respect to a particular component is used herein to mean that the particular component is not purposefully formulated into the composition and/or is present only as a contaminant or in trace amounts. Thus, the total amount of a specific component resulting from any accidental contamination of the composition is less than 0.05%, preferably less than 0.01%. Most preferred are compositions wherein the amount of a particular component is undetectable by standard analytical methods.

As used in the specification, "a" or "an" may refer to one or more. As used in the claims, when used in conjunction with the word "comprising", the word "a" or "an" can mean one or more than one.

The term "or" as used in the claims in intended to mean "and/or" unless explicitly stated to refer only to alternatives or the alternatives are mutually exclusive, although the present disclosure supports definitions referring only to alternatives as well as "and/or". As used herein, "another" may mean at least a second or more.

Throughout the present application, the term "about" or "approximatedly" is used to indicate that a value includes the inherent variations due to measurement error, the method employed for determining the value, or variations among study subjects.

In a first aspect, the present application provides the use of a positron myocardial fatty acid metabolism imaging agent and a positron myocardial glucose imaging agent ¹⁸F-FDG to prepare a medicament for PET combined imaging.

In one specific embodiment, provided is the use of a positron myocardial fatty acid metabolism imaging agent and a positron myocardial glucose imaging agent ¹⁸F-FDG for PET combined imaging, wherein,
the chemical formula of the positron myocardial fatty acid metabolism imaging agent is as follows:

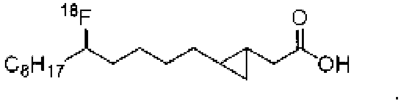

In the context of the specification, the compound is referred to as XTR003.

The chemical formula of the positron myocardial glucose imaging agent ¹⁸F-FDG is:

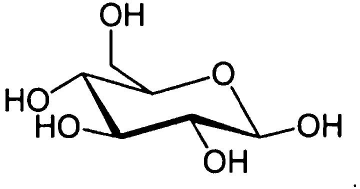

In the context of the specification, the positron myocardial metabolic imaging method is an effective method for assessing viable myocardium. A common practice is to inject ¹⁸F-FDG for myocardial metabolic imaging after glycemic control. However, this method is associated with prolonged imaging duration (1-4 hours) and frequent failure to obtain adequate image quality in patients with insulin resistance, and hypoglycemia events often occur during the glycemic control process, resulting in limited clinical utility. The present application innovatively proposes a protocol in which subjects undergo combined ¹⁸F-FDG and positron myocardial fatty acid metabolism imaging under fasting conditions, thereby replacing the current practice of glycemic-controlled ¹⁸F-FDG myocardial metabolic imaging. Herein, myocardial energy metabolism includes glucose metabolism and fatty acid metabolism. Blood glucose-regulated ¹⁸F-FDG myocardial metabolic imaging converts myocardial fatty acid metabolism into a glucose-only metabolism to present the energy metabolism of the entire myocardium by regulating blood glucose. ¹⁸F-FDG and positron myocardial fatty acid metabolism combined imaging can simultaneously display the total energy metabolism of the myocardium through (glucose metabolism + fatty acid metabolism).

In one embodiment, the aforementioned use is provided, wherein the PET combined imaging is applied to a mammal.

In another embodiment, the aforementioned use is provided, wherein, the positron myocardial fatty acid metabolism imaging agent and the positron myocardial glucose imaging agent ¹⁸F-FDG are administered to the subject on the same day,
during administration, the subject is first administered with ¹⁸F-FDG myocardial glucose imaging agent for imaging, followed by administration of the positron myocardial fatty acid metabolism imaging agent for imaging. Imaging is performed approximately one hour after ¹⁸F-FDG injection. After imaging is completed, the positron myocardial fatty acid metabolism imaging agent is immediately injected and imaging is performed immediately thereafter.

In another specific embodiment, the aforementioned use is provided, wherein the positron myocardial fatty acid metabolism imaging agent and the positron myocardial glucose imaging agent ¹⁸F-FDG are administered to the subject on the same day.

The subjects are first administered with positron myocardial fatty acid metabolism imaging agent for imaging, followed by administration of the ¹⁸F-FDG myocardial glucose imaging agent for imaging. Imaging is performed immediately after the injection of the positron myocardial fatty acid metabolism imaging agent. Immediately after the imaging was completed, ¹⁸F-FDG myocardial glucose imaging agent was injected. Imaging was performed nearly one hour after the injection.

The subject needs to fast for at least 4 hours before sequentially administering the two imaging agents.

In the context of the specification, the term "administration" may refer to various means of drug intake aimed at introducing the drug into the human blood circulation, allowing it to reach sufficient concentration at specific organs or sites and exert imaging effects before being metabolized and cleared. The "administration" can be performed orally or via intravenous injection. When administered orally, the imaging agent may be in the form of tablets, powders, or oral solutions; when administered intravenously, the imaging agent may be in the form of injectable solutions.

In another specific embodiment, the above-mentioned use is provided, wherein, when administered to a subject on the same day, the subject is first administered the ¹⁸F-FDG myocardial glucose imaging agent for imaging, followed by administration of the positron myocardial fatty acid metabolism imaging agent for imaging.

The ratio of the injection dose (unit: mCi or MBq) of the positron myocardial glucose imaging agent ¹⁸F-FDG to that of the positron myocardial fatty acid metabolism imaging agent is 1:x, where x≥1; preferably 1:2-4, and most preferably 1:3. As an example, the value of x may be 2.0, 2.2, 2.4, 2.6, 2.8, 3.0, 3.2, 3.4, 3.6, 3.8, or 4.0.

In the context of the specification, the value of x may be infinite, but in actual use, values of x between 2 and 4 are most frequently used. This range is selected because it achieves a relatively optimal efficacy-cost ratio. When x exceeds 4, although the imaging effect gradually improves, the degree of improvement does not exhibit a linear relationship with the injection dose.

In the context of the specification, the value of x must be greater than 1; values less than 1 would result in excessive image noise, leading to failure in image visualization.

In one embodiment, the above-mentioned use is provided, wherein, when administered to a subject on the same day, the subject is first administered with a positron myocardial fatty acid metabolism imaging agent for imaging, followed by administration of a ¹⁸F-FDG myocardial glucose imaging agent for imaging.

The ratio of the injection dose (unit: mCi or MBq) of the positron myocardial fatty acid metabolism imaging agent to that of the ¹⁸F-FDG myocardial glucose imaging agent is 1:x, wherein x≥1; preferably 1:2-4; most preferably 1:3. As an example, the value of x may be 2.0, 2.2, 2.4, 2.6, 2.8, 3.0, 3.2, 3.4, 3.6, 3.8, or 4.0.

In the context of the specification, the value of x may be infinite, but in actual use, values of x between 2 and 4 are most frequently used. This range is selected because it achieves a relatively optimal efficacy-cost ratio. When x exceeds 4, although the imaging effect gradually improves, the degree of improvement does not exhibit a linear relationship with the injection dose.

In the context of the specification, the value of x must be greater than 1. Because ¹⁸F-FDG and XTR003 are both ¹⁸F-labeled PET imaging agents, when the value of x is less than 1, the myocardial uptake of the first injected subject will interfere with the signal of the subsequently injected subject, affecting the accuracy of the PET imaging for the latter.

In yet another specific embodiment, the above use is provided, wherein the positron myocardial fatty acid metabolism imaging agent and the positron myocardial glucose imaging agent ¹⁸F-FDG are administered to the subject on two separate days.

During administration, the subject is administered with ¹⁸F-FDG myocardial glucose imaging agent for imaging on the first day, followed by administration of the positron myocardial fatty acid metabolism imaging agent for imaging on the second day.

The subject needs to fast for at least 4 hours before administering the imaging agent on two separate days.

In another specific embodiment, the above use is provided, wherein the positron myocardial fatty acid metabolism imaging agent and the positron myocardial glucose imaging agent ¹⁸F-FDG are administered to the subject on two separate days.

During administration, the subject is administered with positron myocardial fatty acid metabolism imaging agent for imaging on the first day, followed by administration of the ¹⁸F-FDG myocardial glucose imaging agent for imaging on the second day;

The subject needs to fast for at least 4 hours before administering the imaging agent on two separate days.

In the context of the specification, the administration of the imaging agent twice a day and on two separate days are also referred to as "one-day method" and "two-day method", respectively. The terms "one-day method" and "two-day method" are general concepts well known in the field of imaging agents.

In another specific embodiment, the above-mentioned use is provided, wherein, the PET image of the PET combined imaging is used in combination with the myocardial perfusion image to assess the viable myocardium and scarred myocardium in the infarcted area, and the myocardial perfusion image is obtained from SPECT or PET myocardial perfusion imaging.

In the context of the specification, PET is the abbreviation of "Positron Emission Computed Tomography"; it is a relatively advanced clinical imaging technology in the field of nuclear medicine. The general method of PET involves labeling a substance (generally one essential for biological metabolism, such as glucose, proteins, nucleic acids, or fatty acids) with a short-lived radionuclide (such as ¹⁸F, ¹¹C, etc.). After injection into the human body, the accumulation of the substance in metabolism is used to reflect the status of biological metabolic activities, thereby enabling diagnostic purposes. The main substance used in hospitals is fluorodeoxyglucose, abbreviated as FDG. Its mechanism is based on the varying metabolic states of different human tissues. Malignant tumor tissues, which exhibit high metabolic activity, demonstrate enhanced glucose metabolism and consequently greater tracer accumulation. These characteristics can be reflected through images, thereby enabling the diagnosis and analysis of lesions. In the context of the specification, "myocardial perfusion imaging" is a non-invasive examination for heart diseases that was initially developed in the 1970s. After nearly 40 years of development, its great diagnostic value has been widely accepted worldwide and has become an important imaging method for the diagnosis, efficacy evaluation and prognosis of coronary heart disease. Myocardial perfusion imaging includes single photon emitter and positron emission tomography (PET) myocardial perfusion imaging. Currently, single-photon emission computed tomography (SPECT) is commonly used in clinical practice. Commonly used myocardial perfusion imaging agents are thallium-201 (TI-201), technetium-99m-methoxyisobutylisonitrile (Tc-99m-MIBI), and technetium-99m-tetrofosmin.

In a second aspect, the present application provides an imaging agent kit.

In one embodiment, an imaging agent kit is provided, comprising a positron myocardial fatty acid metabolism imaging agent and a positron myocardial glucose imaging agent ¹⁸F-FDG;
the chemical formula of the positron myocardial fatty acid metabolism imaging agent is as follows: 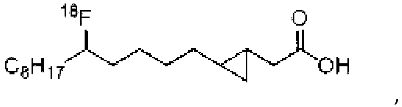
the chemical formula of the positron myocardial glucose imaging agent ¹⁸F-FDG is:

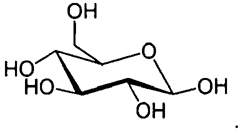

Based on the above uses, the kit provides an amount sufficient for at least a single administration for a 75 kg human, wherein the injection dose ratio (unit: mCi or MBq) between the positron myocardial fatty acid metabolism imaging agent and the ¹⁸F-FDG myocardial glucose imaging agent is 1:2-5, and the absolute injection dose is, for example, 1mCi:2-5mCi; or the injection dose ratio (unit: mCi or MBq) between the ¹⁸F-FDG myocardial glucose imaging agent and the positron myocardial fatty acid metabolism imaging agent is 1:2-5; the absolute injection dose is, for example, 1mCi:2-5mCi.

### Examples

### Example 1

Each experiment used a Bama miniature pig approximately one year old, and the experiment was repeated three times. Taking the second of the three experiments as an example: the piglet weighed approximately 10 kg. A constrictive ring was placed at the site of the left anterior descending coronary artery to gradually induce chronic myocardial infarction in the anterior wall myocardium. Four weeks later, for comparative purposes, a ^{99m}Tc-Sestamibi SPECT myocardial perfusion imaging was performed in the morning (this imaging is the gold standard for assessing viable and scarred myocardium in the infarcted area and was used in this study as a reference for comparing PET imaging results). In the afternoon, a one-day protocol for combined ¹⁸F-FDG and XTR003 PET imaging was performed (with at least 2-hour interval from the SPECT conducted in the morning, during which fasting was maintained). ¹⁸F-FDG PET imaging was performed first, followed by XTR003 PET imaging. The injection doses of the above two agents were 1mCi and 2.5mCi, respectively. XTR003 was injected 1 hour after the ¹⁸F-FDG injection, followed immediately by PET imaging.

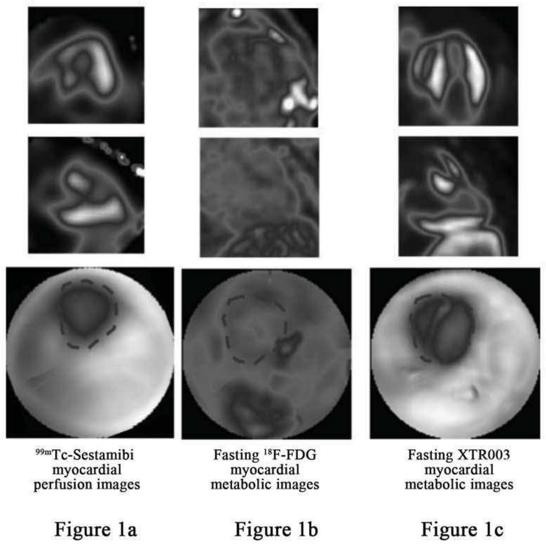

SPECT myocardial perfusion images and the results of the combined ¹⁸F-FDG and XTR003 PET imaging are shown in Figs. 1a-1c. Image interpretation:
Fig. 1a. 99mTc-Sestamibi myocardial perfusion image: A small area of decreased myocardial perfusion is observed in the apical segment to the mid-segment of the anterior wall, represents the myocardial infarction zone (as indicated by the dashed box).
Fig. 1b. Fasting ¹⁸F-FDG myocardial metabolism image: The corresponding myocardial infarction area exhibits low ¹⁸F-FDG uptake, suggesting a state of low glucose metabolism within the infarcted myocardium (as indicated by the dashed box);
Fig. 1c. Fasting XTR003 myocardial metabolic image: moderate uptake of XTR003 persists in the corresponding myocardial infarction area, indicating a moderate level of fatty acid metabolism in the infarced region (as indicated by the dashed box). Comparison among Fig. 1a, Fig. 1b and Fig. 1c shows that viable myocardium remains in the small infarcted area extending from the apical segment to the middle segment of the anterior wall.

### Example 2

Each experiment used a Bama miniature pig about one year old, and the experiment was repeated three times. Taking the second of the three experiments as an example: the piglet weighed approximately 11 kg. A constriction ring was placed at the site of the left anterior descending coronary artery to gradually induce chronic myocardial infarction in the anterior wall myocardium. Three weeks later, for comparative purposes, a 99mTc-Sestamibi SPECT myocardial perfusion imaging was performed in the morning. In the afternoon, a one-day protocol combining ¹⁸F-FDG and XTR003 PET imaging was performed, ¹⁸F-FDG PET imaging was performed first, followed by XTR003 PET imaging. The injection doses of the above two agents were 1mCi and 3.1mCi, respectively. Among them, XTR003 was injected 1 hour after the ¹⁸F-FDG injection, followed immediately by PET imaging.

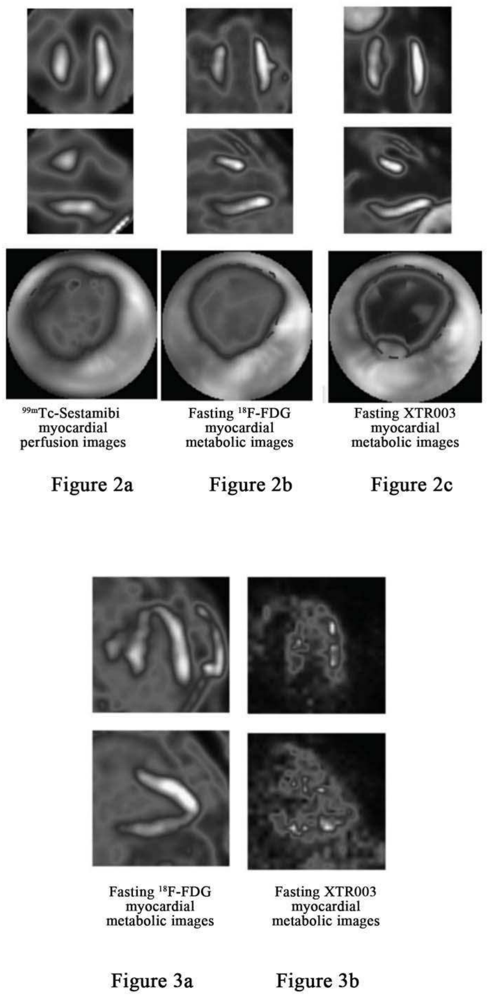

SPECT myocardial perfusion images and the results of the combined ¹⁸F-FDG and XTR003 PET imaging are shown in Figs. 2a-2c. Image interpretation:
Fig. 2a. 99mTc-Sestamibi myocardial perfusion images: A large area of decreased myocardial perfusion is observed from the apical to basal segments, indicating the myocardial infarction zone (as outlined);
Fig. 2b. Fasting ¹⁸F-FDG myocardial metabolism images: There is no ¹⁸F-FDG uptake in the corresponding myocardial infarction area, indicating a state of absent glucose metabolism within the infarcted myocardium (as outlined);
Fig. 2c. Fasting XTR003 myocardial metabolism images: no XTR003 uptake is observed in the corresponding myocardial infarction area, indicating the absence of fatty acid metabolism in the infarcted myocardium (as outlined);

Comparison among Figs. 2a, 2b, and 2c shows that a large-scale myocardial infarction area extending from the apical to basal segments of the anterior wall is necrotic myocardium, which has already progressed to scar myocardium.

### Comparative Example 1. Study: Imaging effect of sequential ¹⁸F-FDG PET imaging followed by XTR003 PET imaging ( injection ratio: 1:x, and x is less than 1)

A disease animal model was constructed as described in Example 1. After myocardial perfusion imaging, "a one-day protocol combining ¹⁸F-FDG PET and XTR003 PET imaging" was performed, wherein XTR003 was injected one hour after the injection of¹⁸F-FDG, followed immediately by PET imaging. The only difference between the Comparative Example and Example 1 is that the injection doses of the above two agents were 1 mCi and 0.25 mCi, respectively. The results of the combined ¹⁸F-FDG and XTR003 PET imaging (also used the second experiment out of three replicates as an example) are shown in Figs. 3a-3b. In the Figs.:
Fig. 3a. Compared with Fig. 1b, the fasting ¹⁸F-FDG myocardial metabolic images show normal image quality.
Fig. 3b. Compared with Fig. 1c, myocardial metabolic images with XTR003 under fasting conditions show excessive image noise due to an insufficient injection dose ratio of XTR003, resulting in imaging failure.

### Example 3. Study: the imaging effect was equally good, with the XTR003 PET imaging first followed by ¹⁸F-FDG PET imaging.

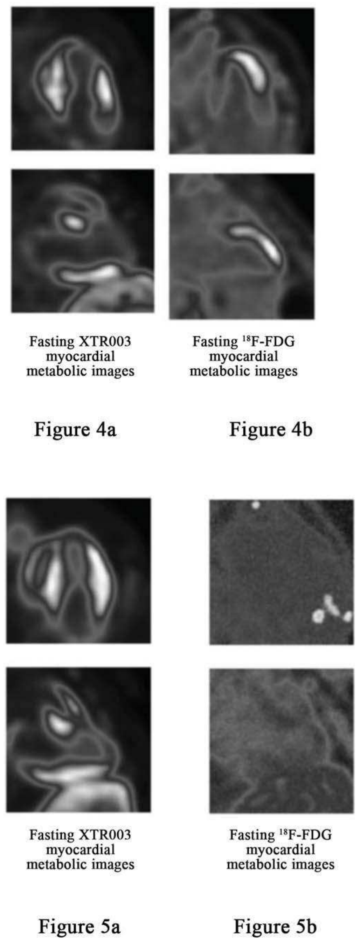

An animal model of the disease was constructed as described in Example 1, differing only in the order of imaging; specifically, XTR003 PET imaging was performed first, followed by ¹⁸F-FDG PET imaging. The injection doses of the two agents were 1 mCi and 3 mCi, respectively. The results of the PET combined imaging with XTR003 and ¹⁸F-FDG are shown in Figs. 4a-4b. In the Figs.:
Fig. 4a. Compared with Fig. 1c, the fasting XTR003 myocardial metabolic images show normal image quality;
Fig. 4b. Compared with Fig. 1b, the fasting ¹⁸F-FDG myocardial metabolic images show normal image quality.

### Comparative Example 2. Study: Imaging Effect of performing XTR003 PET Imaging followed by ¹⁸F-FDG PET Imaging (injection ratio: 1:x, and x is less than 1)

An animal model of the disease was constructed as described in Example 3. After myocardial perfusion, "a one-day protocol combining XTR003 PET and ¹⁸F-FDG PET imaging" was performed, wherein ¹⁸F-FDG was injected one hour after the injection of XTR003, followed immediately by PET imaging. The only difference between the Comparative Example and Example 3 was that the injection doses of the above two agents were 1 mCi and 0.25 mCi, respectively. The results of the combined XTR003 and ¹⁸F-FDG PET imaging are shown in Figs. 5a-5b. In the Figs.:
Fig. 5a. Compared with Fig. 4a, myocardial metabolic images with XTR003 under fasting conditions show normal image quality.
Fig. 5b. Compared with Fig. 4b, the fasting ¹⁸F-FDG myocardial metabolic images show excessive image noise due to an insufficient injection dose ratio of ¹⁸F-FDG, resulting in imaging failure.

### Example 4. Study: Imaging Effect of the "Two-Day Protocol"

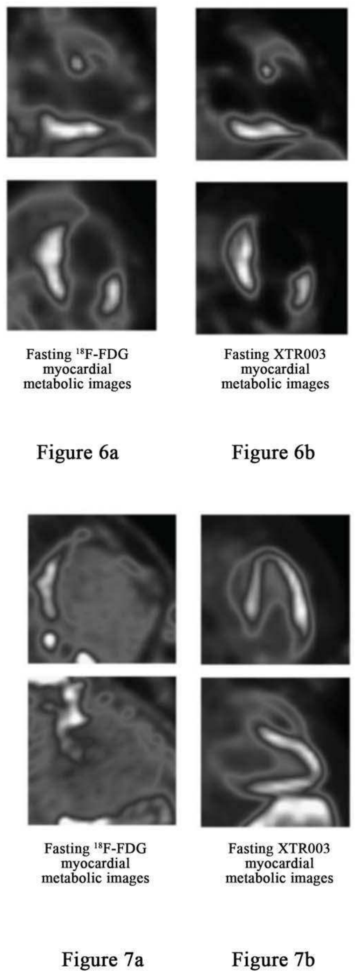

The disease animal model was constructed according to the description of Example 1. The only difference from Example 1 was that after the myocardial perfusion imaging, a "two-day protocol combining ¹⁸F-FDG PET and XTR003 PET imaging" was performed instead of the "one-day protocol". The results of the combined ¹⁸F-FDG and XTR003 PET imaging are shown in Figs. 6a-6b. In the Figs.:
Fig. 6a. Compared with Fig. 1b, the fasting ¹⁸F-FDG myocardial metabolic images show normal image quality.
Fig. 6b. Compared with Fig. 1c, the fasting XTR003 myocardial metabolic images show normal image quality.

### Comparative Example 3. Study: the applicability of "using a single imaging agent without combination"

Each experiment was performed using a Bama miniature pig of about one year old, and the experiment was repeated three times; taking the second experiment of the three experiments as an example: the piglet weighed about 10 kg, and the two imaging agents were administered separately for imaging rather than in combination. The results of separate PET imaging with ¹⁸F-FDG and XTR003 are shown in Figs. 7a and 7b, respectively. In the Figs.:
Fig. 7a. Compared with Fig. 1b, the fasting ¹⁸F-FDG myocardial metabolic images are not visible.
Fig. 7b. Compared with Fig. 1c, the fasting XTR003 myocardial metabolic images show normal image.

Due to low glucose metabolism rate (10%-30%) and high fatty acid metabolism rate (60%-90%) in normal myocardium, ¹⁸F-FDG hardly shows any image, while XTR003 can show normal image.

### Comparative Example 4. Study: Imaging effect of ¹⁸F-FDG PET imaging followed by XTR003 PET imaging (injection ratio: 1:x, with x > 4)

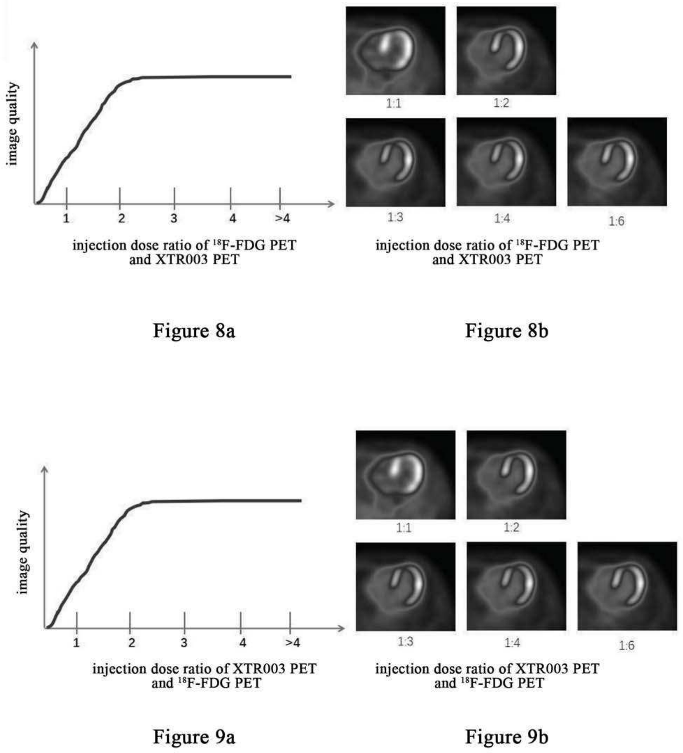

An animal model of the disease was constructed as described in Example 1. After myocardial perfusion imaging, a "a one-day protocol combining ¹⁸F-FDG PET and XTR003 PET imaging" was performed, wherein XTR003 was injected one hour after the injection of ¹⁸F-FDG, followed immediately by PET imaging. The only difference between this Comparative Example and Example 1 lies in the injection doses of the above two agents, which were 1 mCi and 1, 2, 3, 4, and 6 mCi, respectively. The results of the PET combined imaging of ¹⁸F-FDG PET and XTR003 (also using the second experiment of three repetitions as an example) are shown in Figs. 8a-9b. In the Figs.:
Fig. 8a shows the changes in image quality with varying ratios of the two agents, ¹⁸F-FDG PET and XTR003 PET.
Fig. 8b shows actual images corresponding to different ratios of the two agents, ¹⁸F-FDG PET and XTR003 PET.

### Comparative Example 5. Study: Imaging effect of sequential PET imaging with XTR003 followed by ¹⁸F-FDG (injection ratio: 1:x, with x > 4)

An animal model of the disease was constructed as described in Example 3. After myocardial perfusion, "a one-day protocol combining XTR003 PET and ¹⁸F-FDG PET imaging" was performed, wherein ¹⁸F-FDG was injected one hour after the injection of XTR003, followed immediately by PET imaging. The only difference between the Comparative Example and Example 3 was that the injection doses of the two agents were 1 mCi and 1, 2, 3, 4, and 6 mCi, respectively. The results of the combined XTR003 PET and ¹⁸F-FDG imaging are shown in Figs. 9a-9b. In the Figs.:
Fig. 9a shows changes in image quality with varying ratios of the two agents, XTR003 PET and ¹⁸F-FDG PET.
Fig. 9b shows actual images corresponding to different ratios of the two agents, XTR003 PET and ¹⁸F-FDG PET.

It should be noted that when using the "one-day protocol" for imaging, a higher dosage of the subsequently administered imaging agent yields better imaging results; however, increasing the injection dose ratio to >4 does not further improve image quality. On the contrary, excessive dosage may lead to a significant increase in the cost of the imaging agent and elevate radiation risk for the patient. Experiments have shown that an optimal imaging effect is achieved when the ratio of the first-administered imaging agent to the subsequently administered imaging agent ranges between 1:2 to 1:4.

Although the embodiments of the present application are described above in conjunction with the accompanying drawings, the present application is not limited to the above-mentioned specific embodiments and application fields. The above-mentioned specific embodiments are intended to be illustrative and instructive, rather than restrictive. Those skilled in the art may make various modifications guided by the specification and without departing from the scope of claims of the present application, all of which shall fall within the protection scope of the present application.

## Claims

1. Use of a positron myocardial fatty acid metabolism imaging agent and a positron myocardial glucose imaging agent ¹⁸F-FDG for preparing a medicament for PET combined imaging, wherein,
the chemical formula of the positron myocardial fatty acid metabolism imaging agent is as follows: 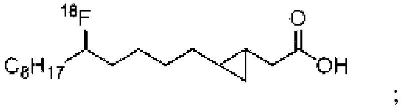
the chemical formula of the positron myocardial glucose imaging agent ¹⁸F-FDG is:

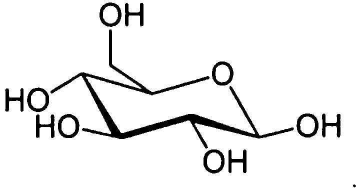

2. The use according to claim 1, wherein the PET combined imaging is applied to a mammal.

3. The use according to claim 2, wherein
the positron myocardial fatty acid metabolism imaging agent and the positron myocardial glucose imaging agent ¹⁸F-FDG are administered to a subject on the same day,
during administration, the subject is first administered with the myocardial glucose imaging agent ¹⁸F-FDG for imaging, followed by the administration of the positron myocardial fatty acid metabolism imaging agent for imaging, imaging is performed approximately one hour after the injection of the ¹⁸F-FDG, immediately after completion of this imaging, the positron myocardial fatty acid metabolism imaging agent is injected, and imaging is performedpromptly thereafter,
or
the subject is first administered with the positron myocardial fatty acid metabolism imaging agent for imaging, followed by the myocardial glucose imaging agent ¹⁸F-FDG for imaging, imaging is performed immediately after the injection of the positron myocardial fatty acid metabolism imaging agent; upon completion of this imaging session, the myocardial glucose imaging agent ¹⁸F-FDG is injected, and imaging is performed nearly one hour after the injection;
the subject needs to fast for at least 4 hours before sequentially administering the two imaging agents.

4. The use according to claim 3, wherein, when administered to a subject on the same day, the subject is first administered with the myocardial glucose imaging agent ¹⁸F-FDG for imaging, followed by the positron myocardial fatty acid metabolism imaging agent for imaging,
the ratio of injection dose of the positron myocardial glucose imaging agent ¹⁸F-FDG to that of the positron myocardial fatty acid metabolism imaging agent is 1:x, and x≥1.

5. The use according to claim 4, wherein x is 2 to 4.

6. The use according to claim 3, wherein, when administered to a subject on the same day, the subject is first administered the positron myocardial fatty acid metabolism imaging agent for imaging, followed by administration of the positron myocardial glucose imaging agent ¹⁸F-FDG for imaging,
the ratio of injection dose of the positron myocardial fatty acid metabolism imaging agent to the ¹⁸F-FDG myocardial glucose imaging agent is 1:x, and x≥1.

7. The use according to claim 6, wherein x is 2 to 4.

8. The use according to claim 2, wherein the positron myocardial fatty acid metabolism imaging agent and the positron myocardial glucose imaging agent ¹⁸F-FDG are administered to the subject on two separate days,
during administration, the subject is administered with the ¹⁸F-FDG myocardial glucose imaging agent for imaging on the first day, followed by administration of the positron myocardial fatty acid metabolism imaging agent for imaging on the second day, or
during administration, the subject is administered with the positron myocardial fatty acid metabolism imaging agent for imaging on the first day, followed by administration of the ¹⁸F-FDG myocardial glucose imaging agent for imaging on the second day;
the subject needs to fast for at least 4 hours before administering the imaging agents on two separate days.

9. The use according to claim 2, wherein the PET images of the PET combined imaging are used in conjunction with myocardial perfusion images to assess the viable myocardium and scarred myocardium in the infarcted area, and the myocardial perfusion images are obtained from SPECT or PET myocardial perfusion imaging.

10. An imaging agent kit, comprising a positron myocardial fatty acid metabolism imaging agent and a positron ¹⁸F-FDG G myocardial glucose imaging agent;
the chemical formula of the positron myocardial fatty acid metabolism imaging agent is as follows: 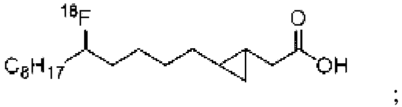
the chemical formula of the positron myocardial glucose imaging agent ¹⁸F-FDG is:
